(19) Europäisches Patentamt
European Patent Office
Office européen des brevets

(11) Publication number: 0 450 900 A1

(12) **EUROPEAN PATENT APPLICATION**

(21) Application number: 91302858.5

(22) Date of filing: 02.04.91

(51) Int. Cl.$^5$: C08G 77/26, C08G 77/388, C07F 7/18

(30) Priority: 02.04.90 JP 87753/90

(43) Date of publication of application: 09.10.91 Bulletin 91/41

(84) Designated Contracting States: DE FR GB

(71) Applicant: SHIN-ETSU CHEMICAL CO., LTD.
6-1, Ohtemachi 2-chome
Chiyoda-ku Tokyo (JP)

(72) Inventor: Kamei, Masanao
12-37, Isobe 3-chome
Annaka-shi, Gunma-ken (JP)
Inventor: Aoki, Hisashi
19-1, Isobe 3-chome
Annaka-shi, Gunma-ken (JP)
Inventor: Ishihara, Toshinobu
1-3 Minato-cho 2-chome
Jouetsu-shi, Niigata-ken (JP)
Inventor: Kubota, Tohru
1-3 Minato-cho 2-chome
Jouetsu-shi, Niigata-ken (JP)

(74) Representative: Stoner, Gerard Patrick et al
Mewburn Ellis 2 Cursitor Street
London EC4A 1BQ (GB)

(54) **Novel siloxane compounds and method for preparing the same.**

(57) Novel N-alkylpyrrolidone-modified siloxane compounds having the characteristics of both silicone and hydrophilic pyrrolidone are of the general formula :

$$R^1_mR^2_nSiO_{(4-m-n)/2}$$

wherein $R^1$ is a monovalent hydrocarbon, hydrocarbonoxy or hydroxyl group, at least one $R^2$ group is an N-alkylpyrrolidone group, the remaining $R^2$ groups being as defined for $R^1$, and m and n are positive numbers within the ranges: $0 < m < 3$, $0 < n < 3$, and $1.8 \leqq m + n \leqq 2.2$. They are prepared by polymerizing an oligomer of a pyrrolidone derivative, a cyclic siloxane, and a siloxane or by addition reaction of an N-allylpyrrolidone to an organohydrogensiloxane. They are useful as fiber treating agents, foam stabilizers, cosmetic additives and the like.

EP 0 450 900 A1

This invention relates to a novel siloxane compound and a method for preparing the same.

## BACKGROUND OF THE INVENTION

Typical of conventional well-known silicones having improved water solubility are polyether-modified silicones, that is, silicones having polyether introduced into their silicone chain. The polyether-modified silicones have the characteristics of both silicone and polyether and are useful fiber treating agents for rendering fibers hydrophilic and/or softening and smoothing fibers. The polyether-modified silicones are also used as foam stabilizers in the manufacture of expanded urethane due to their interfacial properties. In addition, because of their physiological inertness, lustering and emulsifying abilities, the polyether-modified silicones are applied to cosmetic compositions such as foundation and hair treating agents. There is a need for the development of a silicone having a water-soluble group capable of finding a wider variety of applications.

## SUMMARY OF THE INVENTION

An object of the present invention is to provide a new and improved siloxane compound. Another object of the invention is to provide a method for preparing such a siloxane compound.

According to the present invention, there is provided a siloxane compound having the following general compositional formula (1):

$$R^1_mR^2_nSiO_{(4-m-n)/2} \quad (1)$$

wherein $R^1$ is a substituted or unsubstituted monovalent hydrocarbon group having 1 to 20 carbon atoms or a group of the formula: $-OR^3$ wherein $R^3$ is a hydrogen atom or a substituted or unsubstituted monovalent hydrocarbon group having 1 to 8 carbon atoms,

$R^2$ is a group as defined for $R^1$ or a group of the following formula (2):

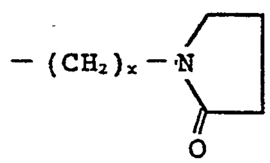

....(2)

wherein x is an integer of from 1 to 8, with the proviso that at least one $R^2$ group is a group of formula (2), and letters m and n are positive numbers within the ranges $0 < m < 3$, $0 < n < 3$, and $1.8 \leqq m + n \leqq 2.2$.

According to a second aspect of the invention, there is provided a method for preparing a siloxane compound as defined above, by polymerizing an oligomer obtained by hydrolysis of a compound of the general formula (3):

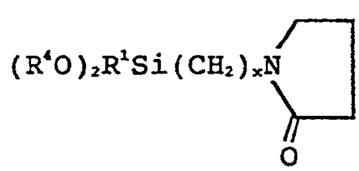

....(3)

wherein $R^1$ is as defined above, $R^4$ is an alkyl group having 1 to 10 carbon atoms, and x is an integer of from 1 to 8, a cyclic siloxane of the general formula (4):

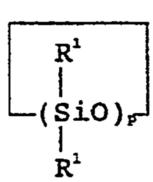

....(4)

wherein $R^1$ is as defined above and p is an integer of at least 2, and a siloxane of the general compositional formula (5).

$$R^1_ySiO_{(4-y)/2} \quad (5)$$

wherein $R^1$ is as defined above and y is a positive number within the range: $0 < y < 3$.

According to a third aspect of the invention, there is provided a method for preparing a siloxane compound as defined above, by effecting addition reaction between an organohydrogensiloxane of the general compositional formula (6):

$$R^1_mR^5_nSiO_{(4-m-n)/2} \qquad (6)$$

wherein $R^1$ is as defined above, $R^5$ is a group as defined for $R^1$ or a hydrogen atom, with the proviso that at least one of $R^5$ groups is a hydrogen atom, and letters m and n are as defined above, and an N-allylpyrrolidone of the general formula (7):

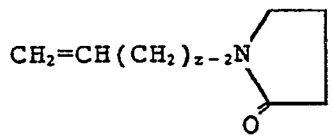

....(7)

wherein z is an integer of from 2 to 8.

The novel siloxane compounds possess the characteristics of both silicone and pyrrolidone because they have an N-alkylpyrrolidone group of formula (2) introduced therein as a water-solubility improving group so that the water solubility of pyrrolidone is imparted to siloxane. Therefore, they are not only useful as fiber treating agents, foam stabilizers, and cosmetic additives like the conventional polyether-modified silicones, but will also find additional applications as silicone fluid having improved heat resistance because of a possibility of comodification with organic groups such as epoxy, amino and carboxyl groups.

DESCRIPTION OF THE PREFERRED EMBODIMENTS

The novel siloxane compounds of the invention are N-alkylpyrrolidone-modified siloxane compounds of formula (1).

$$R^1_mR^2_nSiO_{(4-m-n)/2} \qquad (1)$$

In formula (1), $R^1$ is a substituted or unsubstituted monovalent hydrocarbon group having 1 to 20 carbon atoms or a group of the formula: $-OR^3$ wherein $R^3$ is a hydrogen atom or a substituted or unsubstituted monovalent hydrocarbon group having 1 to 8 carbon atoms,

$R^2$ is a group as defined for $R^1$ or a group of the following formula (2):

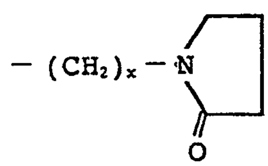

....(2)

wherein x is an integer of from 1 to 8, with the proviso that at least one $R^2$ group is a group of formula (2), and letters m and n are positive numbers within the ranges $0 < m < 3$, $0 < n < 3$, and $1.8 \leqq m + n \leqq 2.2$.

More particularly, examples of the substituted or unsubstituted monovalent hydrocarbon group having 1 to 20 carbon atoms represented by $R^1$ include alkyl groups such as methyl, ethyl, butyl, dodecyl, and octadecyl groups, alkenyl groups such as vinyl and allyl groups, cycloalkyl groups such as cyclohexyl and cycloheptyl groups, aryl groups such as phenyl and naphthyl groups, and substituted ones of these groups in which one or more hydrogen atoms are replaced by halogen atoms or organic groups including alkyl groups such as methyl, ethyl and propyl groups. Examples of the hydrocarbon group having 1 to 8 carbon atoms represented by $R^3$ include those hydrocarbon groups mentioned above, but having 1 to 8 carbon atoms. The groups represented by $-OR^3$ include hydroxyl, alkoxy, alkenyloxy, aryloxy, and acyloxy groups.

In formula (1), $R^2$ is a group as defined for $R^1$ or an N-alkylpyrrolidone group of formula (2).

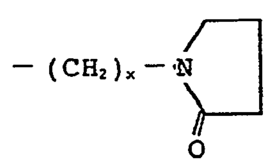

....(2)

In formula (2), x is an integer of from 1 to 8 , preferably equal to 2 or 3. It is essential that the organopolysiloxane contain at least one group of formula (2) in its molecule.

Letters m and n are positive numbers within the ranges $0 < m < 3$, $0 < n < 3$, and $1.8 \leqq m + n \leqq 2.2$, preferably $0.9 \leqq m \leqq 1.1$, $0.9 \leqq n \leqq 1.1$, and $1.9 \leqq m + n \leqq 2.1$.

Several illustrative, non-limiting examples of the siloxane compound of formula (1) are given below.

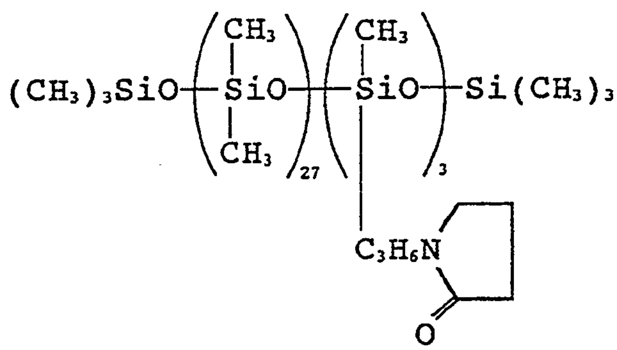

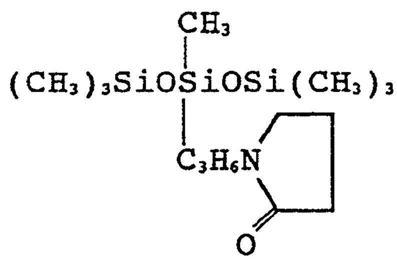

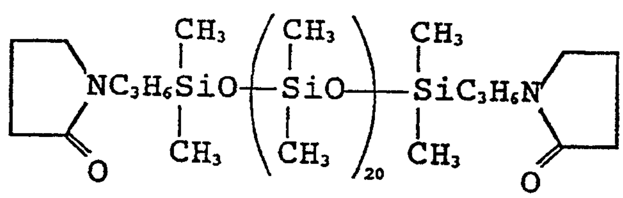

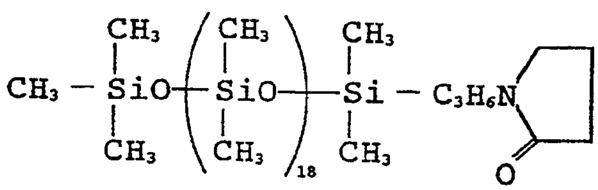

The siloxane compounds of formula (1) can be prepared in two ways.

The first method uses three starting reactants. The first reactant is obtained by subjecting a compound of the general formula (3):

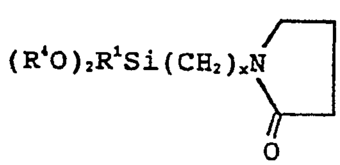

....(3)

to hydrolysis in the presence of water and an alkali or acid catalyst to produce an oligomer having units of the following formula (8):

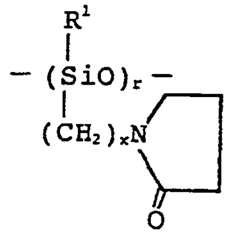 ....(8)

In formulae (3) and (8), $R^1$ is as defined above, $R^4$ is an alkyl group having 1 to 10 carbon atoms, for example, methyl, ethyl, propyl, butyl and pentyl groups, x is an integer of from 1 to 8, and r is an integer of from 3 to 7. The alkali catalysts used herein include inorganic alkalies such as potassium hydroxide and sodium hydroxide and organic alkalies such as triethylamine and pyridine. The acid catalysts used herein include hydrochloric acid, sulfuric acid, and nitric acid. For hydrolysis, the reaction temperature generally ranges from room temperature to about 100°C, preferably from 60 to 80°C and the reaction time generally ranges from about 1 to about 3 hours. At the end of hydrolysis, the reaction solution may be combined with toluene, benzene, xylene, ethanol or the like and the oligomer of formula (8) is then isolated from the mixture by stripping.

The second reactant is a cyclic siloxane of the general formula (4).

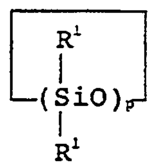 ....(4)

In formula (4), $R^1$ is as defined above and p is an integer of at least 2, preferably 2 to 5, more preferably from 3 to 5.

Examples of the cyclic siloxane of formula (4) include octamethyltetrasiloxane and hexamethyltrisiloxane, to name a few.

The third reactant is a siloxane of the general compositional formula (5).

$$R^1_ySiO_{(4-y)/2} \quad (5)$$

In formula (5), $R^1$ is as defined above and y is a positive number within the range: $0 < y < 3$, preferably from 1.8 to 2.2.

Several illustrative, non-limiting examples of the siloxane of formula (5) are given below.

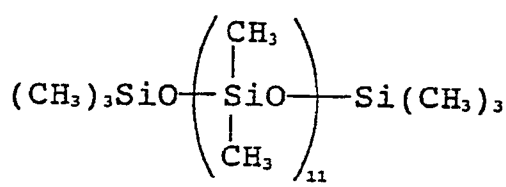

$(CH_3)_3SiOSi(CH_3)_3$

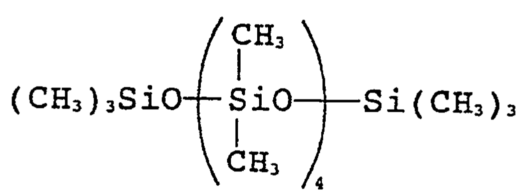

The first method effects polymerization reaction on a mixture of an oligomer resulting from hydrolysis of a compound of formula (3), a cyclic polysiloxane of formula (4), and a polysiloxane of formula (5) in the presence

of an alkaline catalyst, thereby producing a siloxane compound of formula (1).

The alkaline catalysts used for promoting polymerization include cesium hydroxide, potassium hydroxide, sodium hydroxide, and tetrabutyl phosphate. The reaction temperature generally ranges from room temperature to about 150°C, preferably from 110 to 150°C and the reaction time generally ranges from about 3 to about 5 hours. At the end of reaction, the desired siloxane compound of formula (1) is isolated from the reaction mixture by stripping in vacuum.

The second method is to effect addition reaction of an N-allylpyrrolidone of the general formula (7):

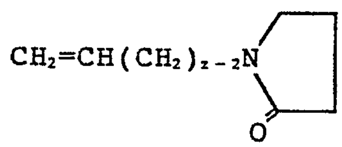 ....(7)

to a hydrogen atom of an organohydrogensiloxane of the general compositional formula (6):

$$R^1_mR^5_nSiO_{(4-m-n)/2} \qquad (6)$$

in the presence of an addition reaction catalyst, thereby preparing a siloxane compound of formula (1) corresponding to formula (6).

In formulae (6) and (7), $R^1$, m and n are as defined above, $R^5$ is a group as defined for $R^1$ or a hydrogen atom, with the proviso that at least one $R^5$ group is a hydrogen atom, and z is an integer of from 2 to 8.

Several illustrative, non-limiting examples of the organohydrogensiloxane of formula (6) are given below.

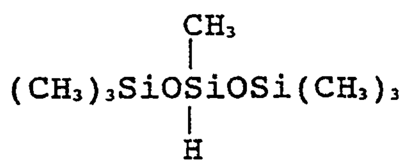

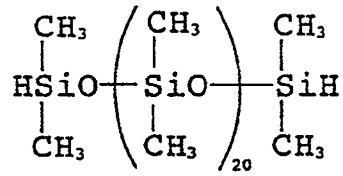

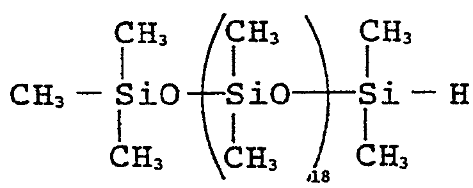

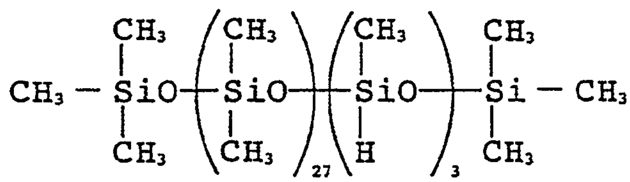

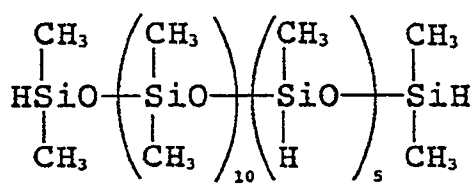

The organohydrogensiloxane of formula (6) and the N-allylpyrrolidone of formula (7) are used in such amounts that the molar ratio of the hydrogen atom in the organohydrogensiloxane to the N-allylpyrrolidone may range from 1:1 to 2:3.

Addition reaction may be effected in a solventless system or in solvents free of active hydrogen, for example, aromatic hydrocarbons such as toluene and xylene, aliphatic hydrocarbons such as hexane and

octane, esters such as ethyl acetate and butyl acetate, and chlorinated hydrocarbons such as carbon tetrachloride and trichloroethane. The catalysts used herein are those catalysts well known to promote addition reaction, for example, platinum, palladium, and rhodium complexes and are used in catalytic amounts. The reaction temperature generally ranges from room temperature to about 150°C, preferably from 60 to 120°C and the reaction time generally ranges from about 8 to about 12 hours. The end of reaction can be readily identified by the disappearance of the organohydrogensiloxane of formula (6) as observed by IR absorption spectroscopy.

There have been described novel siloxane compounds of formula (1) which have hydrophilic N-alkylpyrrolidone introduced therein and thus exhibit the characteristics of both silicone and pyrrolidone. Therefore, they are useful as fiber treating agents for rendering fibers hydrophilic and/or softening and smoothing fibers, foam stabilizers in the manufacture of expanded urethane, and additives to cosmetic compositions such as foundation and hair treating agents because of their physiological inertness, lustering and emulsifying abilities, like the conventional polyethermodified silicones. Furthermore, they will find additional applications because they are susceptible to comodification with organic groups such as epoxy, amino and carboxyl groups.

The methods of the invention ensure simple preparation of organopolysiloxanes of formula (1).

## EXAMPLE

Examples of the present invention are given below by way of illustration and not by way of limitation.

### Example 1

A 1-liter flask equipped with a stirrer and condenser was charged with 300 grams of 3-(2-oxy-1-pyrrolidyl)propylmethyldiethoxy-silane of the following formula.

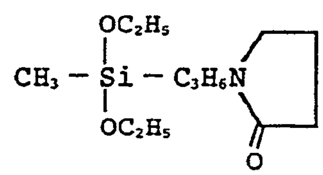

Then 0.05 grams of 35% hydrochloric acid was added to the flask. Using an oil bath, the flask contents were heated to a temperature of 60°C. With stirring, 100 grams of water was added dropwise and hydrolysis effected for 2 hours at 80°C. At the end of reaction, 100 grams of toluene was added to the flask. Vacuum stripping at 100°C yielded 195 grams of a pyrrolidone polymer of the following formula

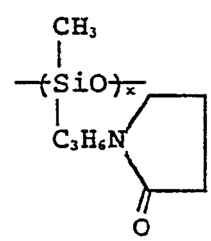

Next, a 200-ml flask was charged with 27.8 grams of the pyrrolidone polymer, 59.3 grams of octamethyltetrasiloxane, and 49.0 grams of dimethylpolysiloxane of the following formula.

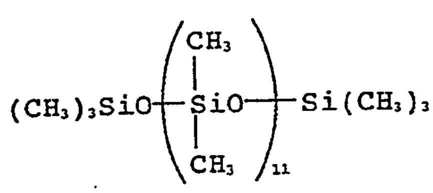

The flask contents were heated to 110°C. Then 0.41 grams of tetrabutyl phosphate was added to the flask and polymerization effected at 110-120°C for 5 hours. The reaction solution was heat treated at 150°C for a further

2 hours and then stripped in vacuum at 130°C, yielding 89 grams of a polysiloxane of the following formula.

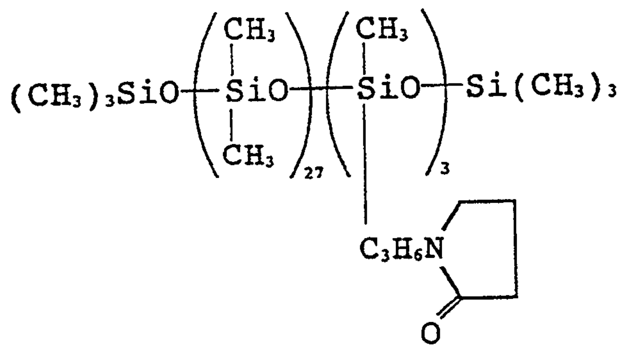

The polysiloxane was subjected to gel permeation chromatography (GPC) and nitrogen analysis, with the following results.

GPC

| | |
|---|---|
| Number average molecular weight (Mn) calculated as polystyrene | 2900 |
| Weight average molecular weight (Nw) calculated as polystyrene | 5500 |
| Degree of multidispersion, Mw/Mn | 1.9 |

Nitrogen analysis

0.12% (calculated: 0.11%)

Example 2

A 500-ml flask equipped with a stirrer and condenser was charged with 224 grams of a hydrogensiloxane of the following average general formula.

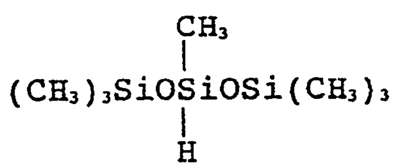

Then 0.30 grams of an isopropyl alcohol solution of 2% chloroplatinic acid was added to the flask. Using an oil bath, the flask contents were heated to a temperature of 90°C. With stirring, 150 grams of N-allylpyrrolidone of the following formula was added dropwise.

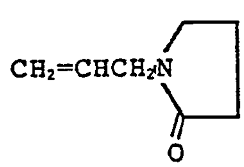

After the completion of addition, the reaction mixture was stirred for 10 hours at 110°C, by which time a peak

indicative of the hydrogensiloxane had disappeared in a gas chromatogram. Distillation of the reaction solution gave 317 grams (yield 91% by weight) of an organopolysiloxane of the following formula as a fraction having a boiling point of 147-149°C/3.5 mmHg.

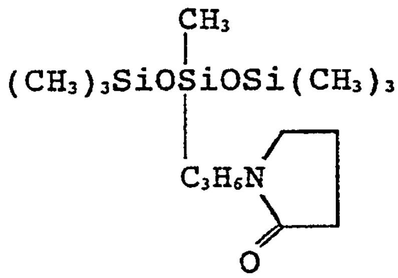

The organopolysiloxane was subjected to proton NMR and GC-MS analysis, with the following results.

$^1$H-NMR

δ (ppm), internal standard: benzene, solvent: $CDCl_3$

0.05 ($Si-CH_3$, s, 21H)
0.5 ($Si-CH_2-$, m, 2H)
1.6 ($-CH_2-$, m, 2H)

2.05 ( 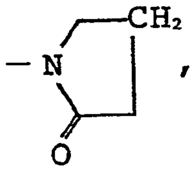 , m, 2H)

2.15 ( 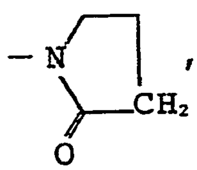 , m, 2H)

3.2 ($-CH_2-N-$, t, 2H)
3.3 ($-CH_2-N-$, t, 2H)

GC-MS analysis

m/e = 349

Example 3

A 500-ml flask equipped with a stirrer and condenser was charged with 161 grams of a both end hydrogen-terminated siloxane of the following average general formula.

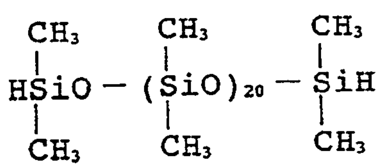

Then 0.05 grams of an isopropyl alcohol solution of 2% chloroplatinic acid was added to the flask. Using an oil bath, the flask contents were heated to a temperature of 90°C. With stirring, 30 grams of N-allylpyrrolidone was added dropwise. The reaction mixture was stirred for 10 hours at 110°C. Substantial disappearance of Si-H

bonds was found as in Example 2. Vacuum stripping of the reaction solution at 110°C yielded 160 grams of an organopolysiloxane of the following formula.

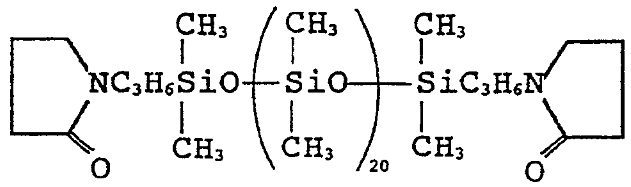

The organopolysiloxane was subjected to GPC and nitrogen analysis, with the following results.

GPC

| | |
|---|---|
| Number average molecular weight (Mn) calculated as polystyrene | 1730 |
| Weight average molecular weight (Mw) calculated as polystyrene | 3100 |
| Degree of multidispersion, Mw/Mn | 1.8 |

Nitrogen analysis

0.11% (calculated: 0.107%)

Example 4

A 500-ml flask equipped with a stirrer and condenser was charged with 296 grams of a single end hydrogen-terminated siloxane of the following average general formula.

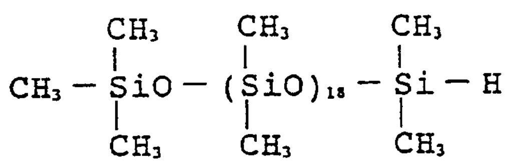

Then 0.1 grams of an isopropyl alcohol solution of 2% chloroplatinic acid was added to the flask. Using an oil bath, the flask contents were heated to a temperature of 90°C. With stirring, 30 grams of N-allylpyrrolidone was added dropwise. The reaction mixture was stirred for 10 hours at 110°C. Substantial disappearance of Si-H bonds was found as in Example 2. Vacuum stripping of the reaction solution at 110°C yielded 285 grams of an organopolysiloxane of the following formula.

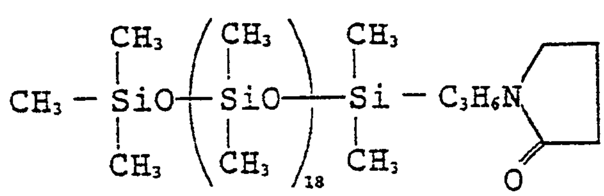

The organopolysiloxane was subjected to GPC and nitrogen analysis, with the following results.

GPC

| | |
|---|---|
| Number average molecular weight (Mn) calculated as polystyrene | 1800 |
| Weight average molecular weight (Mw) calculated as polystyrene | 2150 |
| Degree of multidispersion, Mw/Mn | 1.2 |

Nitrogen analysis

0.065% (calculated: 0.062%)

Although some preferred embodiments have been described, many modifications and variations may be made thereto in the light of the above teachings. It is therefore to be understood that within the scope of the appended claims, the invention may be practiced otherwise than as specifically described.

**Claims**

1. A siloxane compound having the following general compositional formula (1):

$$R^1_mR^2_nSiO_{(4-m-n)/2} \quad (1)$$

wherein $R^1$ is a substituted or unsubstituted monovalent hydrocarbon group having 1 to 20 carbon atoms or a group of the formula:

$$-OR^3$$

wherein $R^3$ is a hydrogen atom or a substituted or unsubstituted monovalent hydrocarbon group having 1 to 8 carbon atoms,

$R^2$ is a group as defined for $R^1$ or a group of the following formula (2):

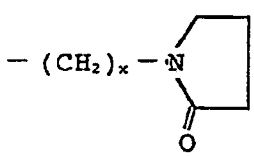

....(2)

wherein x is an integer of from 1 to 8, with the proviso that at least one $R^2$ group is a group of formula (2), and

letters m and n are positive numbers within the ranges $0 < m < 3$, $0 < n < 3$, and $1.8 \leqq m + n \leqq 2.2$.

2. The siloxane compound of claim 1 wherein in formula (1), $R^1$ is methyl.

3. The siloxane compound of claim 1 wherein in formula (2), x is equal to 2 or 3.

4. A method for preparing a siloxane compound as set forth in claim 1, comprising the step of:

polymerizing an oligomer obtained by hydrolysis of a compound of the general formula (3):

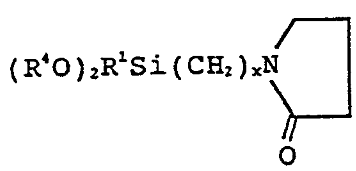

....(3)

wherein $R^1$ is a substituted or unsubstituted monovalent hydrocarbon group having 1 to 20 carbon atoms or a group of the following formula:

$$-OR^3$$

wherein $R^3$ is a hydrogen atom or a substituted or unsubstituted monovalent hydrocarbon group having 1 to 8 carbon atoms, $R^4$ is an alkyl group having 1 to 10 carbon atoms, and x is an integer of from 1 to 8, a cyclic siloxane of the general formula (4):

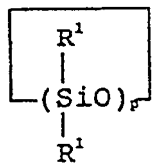

....(4)

wherein $R^1$ is as defined above and p is an integer of at least 2, and a siloxane of the general compositional formula (5):

$$R^1{}_ySiO_{(4-y)/2} \quad (5)$$

wherein $R^1$ is as defined above and y is a positive number within the range: $0 < y < 3$.

5. The method of claim 4 wherein $R^1$ is methyl.

6. The method of claim 4 wherein x is equal to 2 or 3.

7. A method for preparing a siloxane compound as set forth in claim 1, comprising the step of:
effecting addition reaction between an organohydrogensiloxane of the general compositional formula (6):

$$R^1{}_mR^5{}_nSiO_{(4-m-n)/2} \quad (6)$$

wherein $R^1$ is a substituted or unsubstituted monovalent hydrocarbon group having 1 to 20 carbon atoms or a group of the following formula:

$$-OR^3$$

wherein $R^3$ is a hydrogen atom or a substituted or unsubstituted monovalent hydrocarbon group having 1 to 8 carbon atoms, $R^5$ is a group as defined for $R^1$ or a hydrogen atom, with the proviso that at least one $R^5$ group is a hydrogen atom, and letters m and n are positive numbers within the ranges $0 < m < 3$, $0 < n < 3$, and $1.8 \leqq m + n \leqq 2.2$, and an N-allylpyrrolidone of the general formula (7):

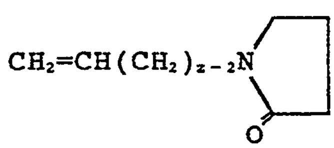

....(7)

wherein z is an integer of from 2 to 8.

8. The method of claim 7 wherein $R^1$ is methyl.

European Patent Office

# EUROPEAN SEARCH REPORT

Application Number: EP 91302858.5

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.5) |
|---|---|---|---|
| X | GB - A - 1 097 453 (DOW CORNING) * Claims 3,6,8; page 2, line 20 - page 3, line 21; examples * | 1-8 | C 08 G 77/26<br>C 08 G 77/388<br>C 07 F 7/18 |
| X | US - A - 3 278 485 (MORGAN et al.) * Examples 2-4,7-11,15-18; claim 5; column 12, lines 37-43 * | 1-8 | |
| A | EP - B1 - 0 131 446 (TORAY SILICONE) * Claim 1 * | 1 | |
| | | | TECHNICAL FIELDS SEARCHED (Int. Cl.5)<br>C 08 G<br>C 07 F |

The present search report has been drawn up for all claims

| Place of search | Date of completion of the search | Examiner |
|---|---|---|
| VIENNA | 10-06-1991 | MARCHART |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)